Europäisches Patentamt

European Patent Office

Office européen des brevets

(11) Publication number: **0 049 632**
**B1**

(12) EUROPEAN PATENT SPECIFICATION

(45) Date of publication of patent specification: 03.07.85

(51) Int. Cl.⁴: **C 12 N 5/00**

(21) Application number: 81304612.5

(22) Date of filing: 05.10.81

(54) Cell culture method.

(30) Priority: 06.10.80 JP 138718/80
08.10.80 JP 139853/80

(43) Date of publication of application:
14.04.82 Bulletin 82/15

(45) Publication of the grant of the patent:
03.07.85 Bulletin 85/27

(84) Designated Contracting States:
AT BE CH DE FR GB LI NL SE

(56) References cited:

BIOLOGICAL ABSTRACTS, vol. 70, 1980, ref.
no. 49413 D.M. FALALEEVA et al.:
"Biosynthesis of alpha-fetoprotein in tissue
culture of human enbryo"
CHEMICAL ABSTRACTS, vol. 94, no. 5, 2nd
February 1981, Page 302, no. 27628p,
Columbus, Ohio, USA P.M. BRADLEY: "Co-
culture of carrot cells and a green alga on
medium deficient in nitrogen"

(73) Proprietor: CHLORELLA INDUSTRY CO., LTD
5th Floor, Toyokuni Building No. 4-6 Shiba
Daimon 2-chome
Minato-ku Tokyo (JP)

(72) Inventor: Maeda, Tadahiko
No. 566-170 Torikoga Dazaifumachi
Chikushi-gun Fukuoka (JP)
Inventor: Tanaka, Kuniaki
No. 345-14, Nagatoishimachi
Kurume-shi Fukuoka, 830 (JP)
Inventor: Katsuta, Hajim
c/o Mikiko Katsuta No. 3-14 Kohinata 1-chome
Bunkyo-ku Tokyo (JP)
Inventor: Takaoka, Toshiko
20-12-115 Kasuga 2-chome
Bunkyo-ku Tokyo (JP)
Inventor: Okuda, Masao
No. 8-4 Taga 1-chome Minami-ku
Fukuoka-shi Fukuoka (JP)

(74) Representative: Perry, Robert Edward et al
GILL JENNINGS & EVERY 53-64 Chancery Lane
London WC2A 1HN (GB)

Courier Press, Leamington Spa, England.

# 0 049 632

**Description**

The present invention relates to a method of cell culture, e.g. of animal or plant cells or tissue.

The technique of so-called "successive cultivation" of animal tissue is known for many biological fields, including medicine and pharmacology, as an effective and practicable means of research. For this reason, the technique has been applied in research, e.g. in elucidating the mechanisms of, for example, cell differentiation and cancer.

For the successive cultivation of animal tissue, it is almost always necessary to use a serum of, for example, an adult, neonate or foetus of a subject which may be bovine or a horse, chicken or rabbit, in addition to a chemically-defined synthetic culture medium comprising, for example, aminoacids, vitamins and/or minerals. The need for a serum is based on the fact that it contains a cell multiplicative growth factor (a substance other than the general nutrient materials); therefore, multiplication of animal tissue cells is impossible without serum. Problems are associated with the use of serum, however; its instability and expense, for example.

When a piece of tissue cut out of a plant body exhibiting relatively rapid growth, such as undifferentiated shoot apex or root cambium, is cultivated in an artificial culture medium, callus formation, which is a phenomenon of undifferentiated cell multiplication, and the formation of differentiated foliate parts and root, are observed separately or simultaneously. In such a plant tissue culture, it is often attempted to enhance growth by the addition of a suitable substance such as coconut milk or yeast extract. The enhancement of growth is limited by the proliferation of the callus, and it has therefore been impossible to produce a differentiated plant body without employing any additional measure. In order to facilitate differentiation, means such as, for example, ultraviolet irradiation and the addition of phytohormones such as indoleacetic acid or benzyladenine have been used. Such means are, however, difficult to control; in particular, synthetic phytohormones may either promote or retard differentiation, depending on the amount which is used.

According to the present invention, a cell culture method comprises cultivating cells in a culture medium comprising microalgae in the form of an aqueous extract.

As used in this specification, the term "microalgae" means unicellular algae or their near relatives, preferred examples of which are Chlorella, Scenedesmus and Spirulina. The algae may be naturally-occurring or cultivated.

For use in the culture medium, the cells of one or more types of microalgae are extracted, e.g. with water itself or an aqueous solution of an acid, base or organic solvent. For extraction, algae cells are brought into contact with an aqueous solvent which may be at ambient or elevated temperature. Hot water extraction is preferred. By way of example, algae cells are suspended in water in an amount of 1 to 1000 grams algae (dry weight) per litre of water and are maintained at 50 to 150°C for 0.5 to 120 minutes, preferably at about 100°C for more than a minute, and are then separated from the water by, for example, centrifugation. The resultant extract may be purified, if desired, by any suitable technique such as gel filtration or dialysis.

The extract usually contains sugars, proteins, polysaccharides and nucleic acids at least, various components having molecular weights in the range of from 1000 to 1,000,000. Such extracts can exhibit multiplication-promoting activity in cultivating animal and plant cells, and the differentiation of plant cells. The term "cells" will be understood herein to include tissue.

The extract can be used per se, as a fraction obtained by molecular weight fractionation thereof, or in the form of a concentrate or dry powder obtained by, for example, freeze-drying or spray-drying. The preferred forms for use in the invention are powders, a high molecular fraction containing sugars, proteins and polysaccharides (especially for animal cells) and a high molecular fraction containing nucleic acids, glycoproteins and polysaccharides (especially for plant cells).

The culture process of the invention may follow the procedures of, and use the same basic culture media as are used in, conventional tissue culture. Thus, a known synthetic culture medium may be used.

For animal cells, the medium preferably contains aminoacids and vitamins at least in addition to the microalgae, and the cultivation is conducted under aseptic conditions. The culture medium may also contain serum, e.g. animal serum, but the amount which is needed may be no more than 10% of that previously considered to be necessary in cultivating animal cells; for example, a culture medium used in the present invention may contain no more than 1% of serum.

For plant cells, the culture medium preferably contains mineral nutrients and a carbon source at least in addition to the microalgae. The medium may also contain, for example, inorganic salts, micro or mineral nutrients, and vitamins. Further, growth-promoting agents such as coconut milk, and differentiation agents such as synthetic phytohormones, may also be included in the medium.

The amount of the microalgae present in the medium is often at least 0.3, and usually no more than 500, mg (dry weight) per liter of the medium. For animal cells, the preferred amount is from 0.3 to 400, and more preferably from 1 to 100, mg/l. For plant cells, the preferred amount is from 1 to 500 mg/l.

Animal cells or tissue which may be cultured by the method of the invention include somatic cells from an animal body, e.g. normal or cancer cells. Plant cells or tissue to be cultured may be from any suitable plant, shoot apex, cambium and hypocotyl of seedlings being preferred. It is also possible to cultivate

2

multiplicated but undifferentiated cells developed in plants, such as callus, and cells or tissue obtained by successive cultivation.

When animal cells are cultivated by the method of the invention, successive cultivation can be maintained by the multiplication promotion effect. The reason for this effect is not clear, but it is theorised that cell metabolism is stimulated by the action of some substance, probably of high molecular weight, such as a glycoprotein or polysaccharide, in the microalgae. This effect has the important advantage that the amount of any serum which is used need be very much less than has previously been the case, thereby reducing costs and facilitating handling. When plant cells such as shoot apex tissue are cultivated by the method of the invention, not only is callus proliferation enhanced, but also differentiation is promoted, so that plant body, stem, leaf or root may be developed from the callus even without callus formation. It is possible to harvest the multiplied mass of cells and the grown body or other parts, or to transplant the differentiated plant body as a seedling. It may also be possible to extract useful materials from callus which may be obtained. Stem or leaf which may have been differentiated exclusively may have important applications. Well replicated/homogeneous seedling cultures may be obtained, allowing conservation of a plant species, for example.

The following Examples illustrate the invention.

Example 1

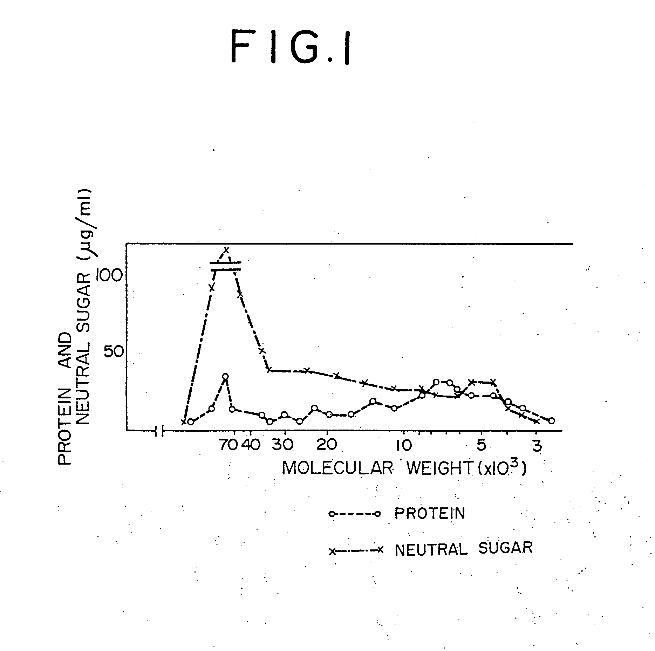

30 g of Chlorella powder were suspended in 1 l of water and subjected to hot water extraction at 100°C for 30 minutes. The suspension was then centrifuged and the supernatant (corresponding to 4.5 g dry extracted matter) was subjected to molecular fractionation on a Sephadex (registered Trade Mark) G-25 column. The fractions with molecular weights above 3,000 were adsorbed on a DEAE-cellulose column, and then eluted with 0.01 M carbonate buffer. A fraction having a component with a molecular weight over 70,000, a component with a molecular weight in the range from 30,000 to 10,000 which comprises rich neutral sugar, and a component with a molecular weight in the range 10,000 to 3,000 which comprises neutral sugar protein in nearly equivalent proportions, was obtained (hereinafter termed Fraction $A_1$). Fraction $A_1$ was freeze-dried to give 0.63 g dry matter. The molecular fractionation chart of fraction $A_1$ is given in Figure 1 of the accompanying drawings.

$5 \times 10^4$ of cell strain RLC-10 (rat liver cells) were cultivated in a culture medium prepared by adding a cattle foetus serum (FCS) in an amount of 10% to a sector (Sector C) of a basic culture medium, DM-160, whose composition is given in Table 1.

TABLE 1
Composition of basic culture medium DM-160

| Amino acids | mg/l | Vitamins | mg/l |
|---|---|---|---|
| Ala | 400 | $B_1$ | 1.0 |
| Arg | 100 | $B_2$ | 1.0 |
| Asp | 25 | $B_6$ | 1.0 |
| Asn | 25 | $B_{12}$ | 0.005 |
| Cys HCl | 80 | Pantothenic acid | 1.0 |
| Glu | 150 | Nicotinamide | 1.0 |
| Gln | 300 | Biotin | 0.1 |
| Gly | 15 | Choline HCl | 5.0 |
| His | 30 | C | 1.0 |
| Ile | 150 | Folic acid | 1.0 |
| Leu | 400 | Inositol | 5.0 |
| Lys | 100 | | |
| Met | 80 | | |
| Phe | 80 | | |
| Pro | 12 | | |
| Ser | 80 | | |
| Thr | 100 | | |
| Trp | 40 | | |
| Tyr | 50 | | |
| Val | 85 | | |

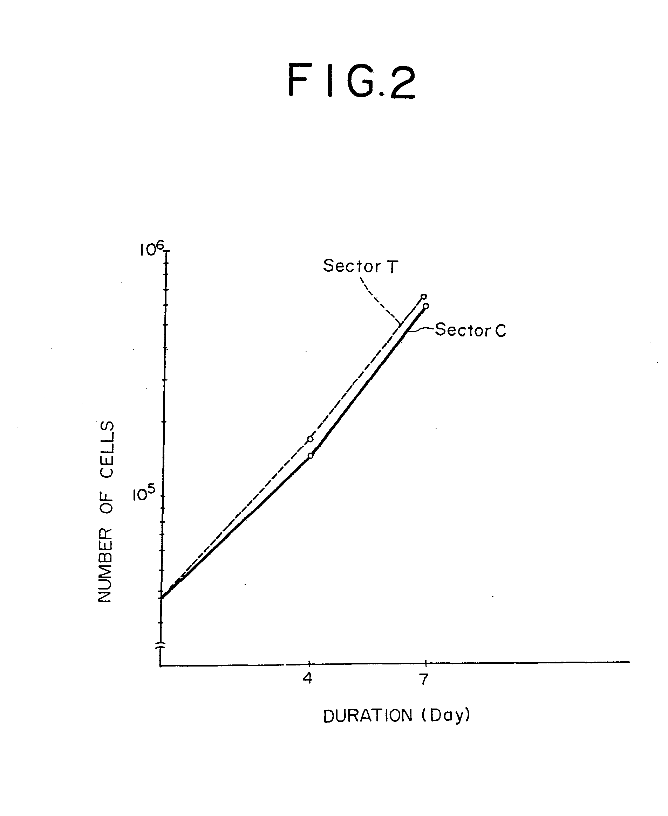

In a similar manner, RLC-10 was cultivated in DM-160 (Sector T), to which had been added 1% FCS and 5 µg/ml Fraction $A_1$. Cell multiplication was observed by comparison of the two cultivation sectors. The sectors showed a similar trend of multiplication of cells. The results obtained are shown graphically in Fig. 2.

Example 2

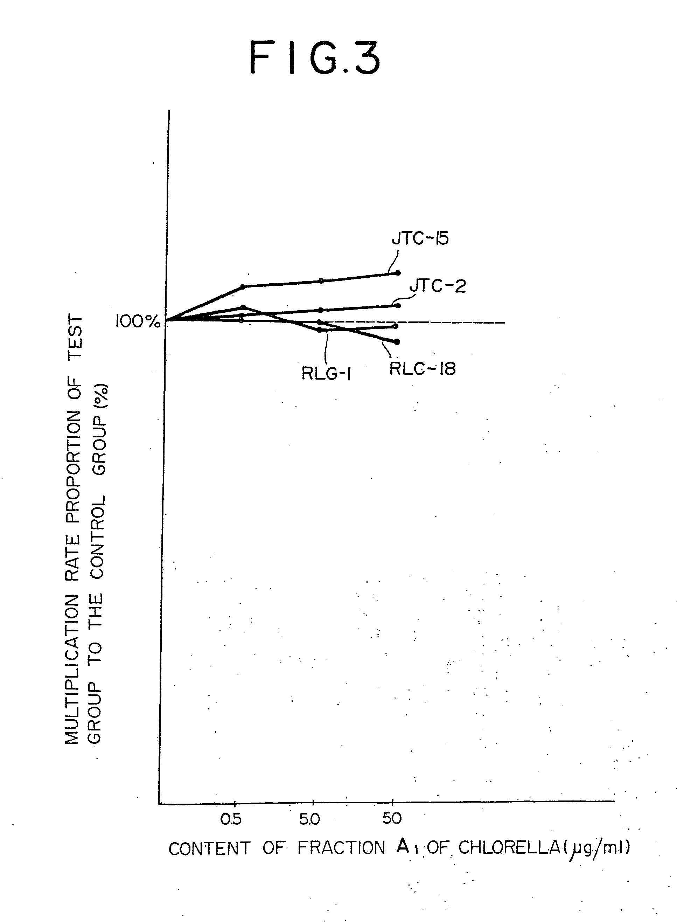

In a similar manner as in Example 1, cell strain JTC-15 (rat ascites hepatoma), JTC-2 (rat ascites hepatoma) RLG-1 (rat lung cells) and RLC-18 (rat liver cells) were examined for their multiplication rates in the medium employed in Example 1 with variable contents, i.e. 0.5 µg/ml, 5 µg/ml and 50 µg/ml, of Fraction $A_1$. The tendencies of the multiplication of the cells were the same. The results obtained are shown graphically in Fig. 3.

Example 3

Shoot apexes (one of the most rapidly growing tissues in a plant body, including a growing point) of carnation were excised and were each placed, in test tubes, on a culture medium. Cultivation was conducted under conditions comprising a 9 hour illumination period (2,000 1×) and a 15 hour dark period per day, at 25°C. The culture medium was the basal culture medium of Murashige & Skoog, the composition of which is given in Table 2, excluding $FeSO_4$ but to which had been added 2.0% sucrose, 0.8% agar-agar and various amounts of a Chlorella extract.

4

TABLE 2
Basal culture medium according to murashige & skoog

| Inorganic salts (mg/l) | | Inorganic salts (mg/l) | | Vitamins etc. (mg/l) | |
|---|---|---|---|---|---|
| $NH_4NO_3$ | 1650 | $H_3BO_3$ | 6.2 | Nicotinic acid | 0.5 |
| $KNO_3$ | 1900 | $MnSO_4 \cdot 4H_2O$ | 22.3 | Pyridoxine-HCl | 0.5 |
| $CaCl_2 \cdot 2H_2O$ | 440 | $ZnSO_4 \cdot 4H_2O$ | 8.6 | Thiamine . HCl | 0.1 |
| $MgSO_3 \cdot 7H_2O$ | 370 | KI | 0.83 | Myoinositol | 100 |
| $KH_2PO_4$ | 170 | $Na_2MoO_4 \cdot 2H_2O$ | 0.25 | Glycine | 2 |
| $Na_2$-EDTA | 37.3 | $CuSO_4 \cdot 5H_2O$ | 0.025 | | |
| $FeSO_4 \cdot 7H_2O$ | 27.8 | $CoCl_2 \cdot 6H_2O$ | 0.025 | | |

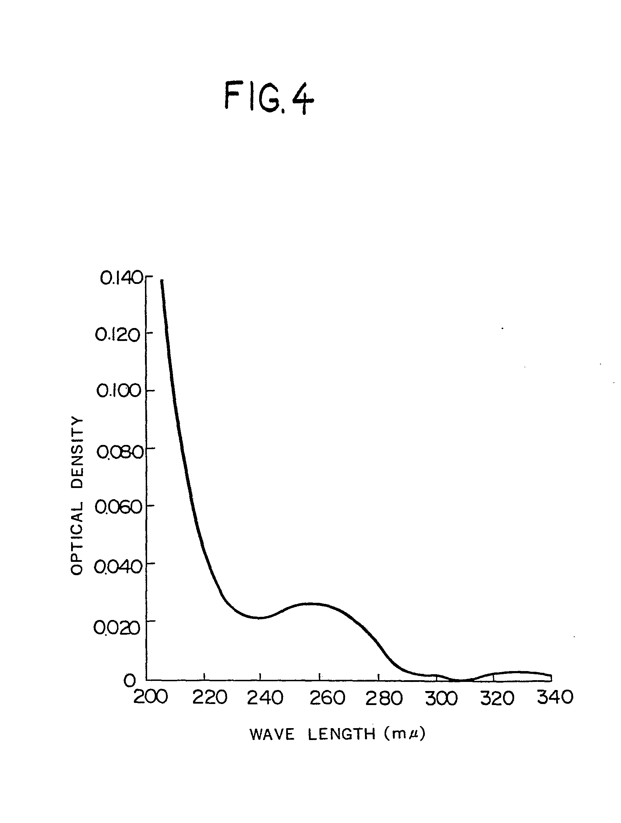

Each culture medium had a pH of 5.5—6.0. The Chlorella extract was obtained by dispersing 30 g of Chlorella dry powder in 1 l of water and subjecting the dispersion to hot water extraction at 100°C for 30 minutes, centrifuging the resulting suspension and freeze-drying the supernatant obtained (4.5 g dry weight). The ultraviolet absorption spectrum of the aqueous solution of this Chlorella extract is given in Fig. 4. The amounts of the extract added to the culture medium were respectively 0, 2, 6 and 20 mg/l.

After cultivation for 2 months, the greatest degree of multiplication and differentiation had occurred in the tissues containing the highest content of the extract.

When the same procedure was repeated using 100 test tube samples for each of the cases in which 0 and 20 mg/l of the extract were added to the medium, the cultured samples were classified as to whether (A) the whole tissue had become callus, without differentiation; (B) stem and leaf were formed with callused stem; (C) stem, leaf and root were formed with callused stem; (D) stem, leaf and root were formed without callus formation; and (E) neither multiplication nor differentiation was observed. Table 3 gives the results of the evaluation for these cultured samples.

TABLE 3

| Added extract (mg/l) | Evaluation | | | | |
|---|---|---|---|---|---|
| | (A) | (B) | (C) | (D) | (E) |
| 0 | 22 | 22 | 11 | 34 | 11 |
| 20 | 15 | 31 | 46 | 8 | 0 |

From these results, a multiplication-promoting effect of the Chlorella extract is observed since the number of (D) and (E) evaluated samples is reduced by the addition of the extract. The relative (A), (B) and (C) figures indicate a differentiation-promoting effect.

Example 4

When naked rice seed is cultivated on an agar-agar culture medium containing 2.0 mg/l of the synthetic growth hormone 2,4-D, callus formation is observed. In this Example, the influence of the addition of microalgae extract upon such callus formation is examined.

Using a basal culture medium B-5 as identified in Table 4, culture media (pH 6.0) were prepared by adding thereto 2.0% by weight of sucrose, 2.0 mg/l of 2,4-D, 0.8% by weight of agar-agar, and the Chlorella extract in amounts varying from 0 to 500 mg/l. The cultivation conditions were the same as in Example 1.

TABLE 4
Basal culture medium B-5 (pH 6.0)

| Component | Content | Component | Content |
|---|---|---|---|
| $NaH_2PO_4 \cdot 2H_2O$ | 171.6 mg/l | $Na_2MoO_4 \cdot 2H_2O$ | 0.2522 mg/l |
| $KNO_3$ | 2527.8 mg/l | $CoCl_2 \cdot 6H_2O$ | 0.0242 mg/l |
| $(NH_4)_2SO_4$ | 132.1 mg/l | KI | 0.7457 mg/l |
| $MgSO_4 \cdot 7H_2O$ | 246.5 mg/l | Nicotinic acid | 1.0 mg/l |
| $CaCl_2 \cdot 2H_2O$ | 111.0 mg/l | Thiamine . HCl | 10.0 mg/l |
| Fe-EDTA | 28 mg/l | Pyridoxine . HCl | 1.0 mg/l |
| $MnSO_4 \cdot H_2O$ | 13.6 mg/l | m-Inositol | 100.0 mg/l |
| $ZnSO_4 \cdot 7H_2O$ | 1.98 mg/l | Sucrose | 20.0 g/l |
| $H_3BO_3$ | 3.03 mg/l | 2,4-D | 2.0 mg/l |
| $CuSO_4$ | 0.0393 mg/l | | |

30 days after the seeds were placed on the culture media, evaluation was made in the same manner as in Example 3. Table 5 shows the results.

TABLE 5

| Added extract (mg/l) | Evaluations | | | | |
|---|---|---|---|---|---|
| | (A) | (B) | (C) | (D) | (E) |
| 0 | 86 | 2 | 3 | 0 | 9 |
| 10 | 54 | 32 | 14 | 0 | 0 |

These results show that the extract promotes differentiation, since the number of samples in classes (B) and (C) are greater in that case. This observation was confirmed, and that a marked effect could be achieved, when the extract was added to the medium in an amount ranging from 1 to 100 mg/l. It was also noted that the number of samples exhibiting cell multiplication was substantially independent of the presence of the extract, although the increase in the weight of the grown body was greater for those samples containing the extract than for those without; this shows that the extract promotes proliferation.

**Claims**

1. A cell culture method, which comprises cultivating cells in a culture medium, characterised in the medium comprises microalgae in the form of an aqueous extract.
2. A method according to claim 1, in which the microalgae are selected from Chlorella, Scenedesmus and Spirulina.
3. A method according to claim 1 or claim 2, in which the cells are animal cells.
4. A method according to claim 3, in which the cells are selected from normal tissue and cancer cells.
5. A method according to claim 3 or claim 4, in which each litre of the medium comprises from 0.3 to 400 mg of the microalgae.
6. A method according to any of claims 3 to 5, in which the medium additionally comprises animal serum.
7. A method according to claim 1 or claim 2, in which the cells are plant cells.
8. A method according to claim 7, in which the cells have been subjected to successive cultivation.
9. A method according to claim 7 or claim 8, in which each litre of the medium comprises from 1 to 500 mg of the microalgae.

# 0 049 632

## Patentansprüche

1. Zellkulturverfahren, welches ein Vermehren von Zellen in einem Kulturmedium umfaßt, dadurch gekennzeichnet, daß das Medium Mikroalgen in Form eines wässerigen Extraktes umfaßt.

2. Verfahren nach Anspruch 1, worin die Mikroalgen unter Chlorella, Scenedesmus und Spirulina ausgewählt sind.

3. Verfahren nach Anspruch 1 oder 2, worin die Zellen tierische Zellen sind.

4. Verfahren nach Anspruch 3, worin die Zellen unter Normalgewebezellen und Krebszellen ausgewählt sind.

5. Verfahren nach Anspruch 3 oder 4, worin jeder Liter des Mediums von 0,3 bis 400 mg der Mikroalgen umfaßt.

6. Verfahren nach einem der Ansprüche 3 bis 5, worin das Medium zusätzlich tierisches Serum umfaßt.

7. Verfahren nach Anspruch 1 oder 2, worin die Zellen pflanzliche Zellen sind.

8. Verfahren nach Anspruch 7, worin die Zellen einer aufeinanderfolgenden Züchtung unterworfen worden sind.

9. Verfahren nach Anspruch 7 oder 8, worin jeder Liter des Medium von 1 bis 500 mg der Mikroalgen umfaßt.

## Revendications

1. Un procédé de culture de cellules, qui consiste à cultiver des cellules dans un milieu de culture, caractérisé en ce que le milieu contient des microalgues sous la forme d'un extrait aqueux.

2. Un procédé selon la revendication 1, dans lequel les microalgues sont choisis parmi Chlorella, Scenedesmus et Spirulina.

3. Un procédé selon la revendication 1 ou 2, dans lequel les cellules sont des cellules animales.

4. Un procédé selon la revendication 3, dans lequel les cellules sont choisies parmi les cellules de tissus normaux et les cellules cancéreuses.

5. Un procédé selon la revendication 3 ou 4, dans lequel chaque litre du milieu contient de 0,3 à 400 mg des microalgues.

6. Un procédé selon l'une quelconque des revendications 3 à 5, dans lequel le milieu contient en outre un sérum animal.

7. Un procédé selon la revendication 1 ou 2, dans lequel les cellules sont des cellules végétales.

8. Un procédé selon la revendication 7, dans lequel les cellules ont été soumises à des cultures successives.

9. Un procédé selon la revendication 7 ou 8, dans lequel chaque litre du milieu contient de 1 à 500 mg des microalgues.

# F I G.I

0 049 632

FIG.2

2

# F I G.3

# FIG. 4